Europäisches Patentamt

European Patent Office

Office européen des brevets

(19)

(11) EP 0 456 636 B1

(12) **EUROPEAN PATENT SPECIFICATION**

(45) Date of publication and mention
of the grant of the patent:
**13.03.1996 Bulletin 1996/11**

(21) Application number: **89908709.2**

(22) Date of filing: **04.08.1989**

(51) Int Cl.[6]: **C07H 15/04**, A23L 1/236,
A61K 7/16

(86) International application number:
**PCT/FI89/00142**

(87) International publication number:
**WO 90/06317 (14.06.1990 Gazette 1990/14)**

(54) **A CRYSTALLINE LACTITOL MONOHYDRATE AND A PROCESS FOR THE PREPARATION THEREOF, USE THEREOF, AND SWEETENING AGENT**

Ein kristallines Lakitolmonohydrat sowie ein Verfahren zu dessen Herstellung, dessen Verwendung sowie Süssstoff daraus.

MONOHYDRATE DE LACTITOL CRISTALLIN, SON PROCEDE DE PREPARATION ET D'UTILISATION, ET AGENT EDULCORANT

(84) Designated Contracting States:
**AT BE CH DE FR GB IT LI LU NL SE**

(30) Priority: **01.12.1988 FI 885588**

(43) Date of publication of application:
**21.11.1991 Bulletin 1991/47**

(73) Proprietor: **XYROFIN OY**
**SF-00240 Helsinki (FI)**

(72) Inventors:
• **HEIKKILÄ, Heikki, Olavi**
**SF-02320 Espoo (FI)**
• **NURMI, Juha, Veikko**
**SF-10330 Pinjainen (FI)**

(74) Representative: **Hansen, Bernd, Dr.rer.nat. et al**
**Hoffmann, Eitle & Partner**
**Patentanwälte**
**Postfach 81 04 20**
**D-81904 München (DE)**

(56) References cited:
**EP-A- 0 039 981          DE-A- 2 945 672**
**DE-B- 2 133 428**

• **INTERNATL. DAIRY FEDERATION BULLETIN, vol. 212, 1987; C.J. BOOY, pp. 62-68**
• **J. Agric. Food Chem., Vol. 27, No. 4, 1979 JOHN A. VAN VELTHUIJSEN: "Food additives derived from lactose: Lactitol and Lactitol Palmitate", see page 680 - page 686 see particularly pages 680-684, inter alia p. 681, left-hand column line 21 from the bottom.**
• **International dairy federation Bulletin, Vol. 212, 1987 CEES J. BOOY: "Lactitol "A new food ingredient" ", see page 62 - page 68.**
• **DEVELOPMENTS IN SWEETENERS-3, Vol. 3, 1987 (London and New York) C.H. DEN UYL. Pages 65-81**
• **Abstract of a paper presented by M.VAN BOMMEL and J.A.KANTERS at the 6th Europ.Cryst. Meeting, Barcelona on July 28, 1980**

Remarks:
The file contains technical information submitted after the application was filed and not included in this specification

## Description

The invention relates to a new crystalline lactitol monohydrate, and a process for the preparation thereof by crystallization from an aqueous solution, the use of the said new crystalline lactitol monohydrate in dietetic products, confectionery, bakery products, cereals, desserts, jams, beverages, chocolate, chewing gum and ice-cream, as well as in costmetic products, and in the manufacture a pharmaceutical , such as tooth paste. The invention also relates to a new sweetening agent resembling sugar, mainly composed of the said new crystalline lactitol monohydrate.

Lactitol is a bulk sweetener which can be used as a total or partial replacement for sucrose, however, its energy content is only about half of that of sucrose, and it does not cause increased blood glucose content; furthermore, it is tooth-friendly (see Developments in Sweeteners, Ed. Grenby, T.H., Vol. 3, 1987, p. 65-81).

The preparation of lactitol from lactose has been known for a long time. Industrially, lactitol is prepared analogously with the preparation of sorbitol from glucose by hydrogenation in the presence of a Raney nickel catalyst. An aqueous solution of lactose, typically having a concentration between 30 and 40% by weight due to the poor solubility of lactose, is hydrogenated at 70 to 130°C at a pressure between 30 and 74 atm. The preparation is described in Wolfrom, M.L., Burke, W.J., Brown, K.R. and Rose, R.S., J. Am. Chem. Soc. 60, (1938) p. 571-573.

Crystalline lactitol is reported to occur in anhydrous form (anhydride) as well as in the form of a monohydrate and dihydrate, which forms have been known for a long time with the exception of pure monohydrate. Among the crystal forms of lactitol, lactitol monohydrate is of considerable commercial interest on account of its low hygroscopicity.

In the preparation process mentioned above, lactitol anhydride can be crystallized by adding ethanol to a lactitol solution evaporated to a high concentration. After a crystallization time of one month, the yield of lactitol was 80%, and the melting point of the resulting crystal, which was found to be an anhydride, was between 144 and 146°C.

The crystallization of lactitol dihydrate was presumably mentioned for the first time by Senderens, J.B., Compt. Rend. 170, (1920) p. 47-50. Lactitol solution obtained by hydrogenation was evaporated slowly at room temperature so that crystallization was initiated. The melting point of the resulting product was 78°C, and Senderens mistakenly regarded it as a monohydrate. However, it appears from European Patent 0039981 and Wolfrom, M.L., Hann, R.M. and Hudson, C.S., J. Am. Chem. Soc. 74 (1952) p. 1105 that the product obtained by Senderens was a dihydrate having a moisture content of 9.5%, determined by a Karl Fisher method, and a melting point between 76 and 78°C.

The next attempt to prepare lactitol monohydrate by crystallization was made in 1979; the end product, however, was an impure dihydrate, see van Velthuijsen, J.A., J. Agric. Food Chem. 27, (1979) p. 680. The solubility of the "monohydrate" so obtained was reported to be 64% by weight at 25°C; however, it has been proved that lactitol monohydrate cannot have such a solubility, see the above-mentioned European Patent 0039981. Taking into consideration that the alleged monohydrate was impure (4.5% of other sugars and dihydrate), the corrected solubility is the right solubility of lactitol (about 59% by weight on dry substance basis). The reported melting range from 94 to 97°C also indicates the presence of a slightly overdried (impure) dihydrate.

Another attempt to prepare lactitol monohydrate was made in 1981, see the above-mentioned European Patent 0039981. The low crystallization temperature, however, resulted in the formation of monohydrate and dihydrate either as mixed crystals or separately, the product being then dried into partially anhydrated monohydrate. The reported melting point 121-123°C is that of partially anhydrated monohydrate from which the lattice cell constants of monohydrate are obtained by means of a single-crystal X-ray diffractometer within the measuring accuracy. The surface layer of partially anhydrated monohydrate may be integral (double crystal) or fragmented or it may be composed of numerous separate anhydride crystals, which becomes apparent from the high scattering of the lattice cell constants when determined by the single-crystal method. Partially anhydrated monohydrate is at least as stable as a complete anhydride which does not bind water at room temperature at moderate relative humidities.

The surface layer of said partially anhydrated monohydrate is imperfect and under suitable conditions it will be restored partially or completely to the monohydrate form. Since the formation of a perfect lattice structure is irreversible, the restored crystal structure will never be perfect, i.e. if the anhydride or partially anhydrated monohydrate takes (binds) crystal water, the product obtained does not have the crystal structure of lactitol monohydrate. Both the anhydrated and partially restored monohydrate easily get cloddy and have a rather poor flowability and rather a high hygroscopicity on account of the fragmented surface and high dust content of the product.

Monohydrate loses all of its crystal water as rapidly as in two hours when it is dried at 105°C in a conventional laboratory oven. The melting point of the "monohydrate" disclosed in European Patent 0039981, that is, 121-123°C, corresponds to that of a monohydrate anhydrated to a degree of anhydration of 10 to 15%. In addition, the "monohydrate" disclosed in this patent, which lost 2% of its weight at 130°C during 3 days, is originally a monohydrate anhydrated to a degree of anhydration of 60%. The "monohydrates" disclosed in the European patent Are not monohydrates anhydrated from pure monohydrate; instead, they are overdried products formed from the mixtures of dihydrate and monohydrate due to the crystallization method.

Lactitol hydrate powders anhydrated to a moisture content of less than 3% have been prepared by drying both lactitol solution and crystalline hydrate. The hygroscopicity of these powders is utilized in drying moist mixtures (European

EP 0 456 636 B1

Patent Application 0231643).

The preparation of pure lactitol monohydrate having lattice cell constants a = 7.815 ± 0.008 Å, b = 12.682 ± 0.008 Å, and c = 15.927 ± 0.008 Å; and a melting range between 94 and 100°C, preferably between 94 and 98°C, has now succeeded for the first time. The melting range was determined with a Büchi Tottol melting point apparatus. The lactitol content of the said pure lactitol monohydrate is more than 99.5% on a dry substance basis, and its moisture content is between 4.85 and 5.15%.

The new lactitol monohydrate has a good flowability and long shelf life, and it is stable at room temperatures, in relative humidities ranging from 25 to 75%. After having been stored under varying atmospheric conditions for about two years in an open paper sack, the lactitol monohydrate did not become cloddy and its flowability was 5.1 s/100 g measured by a funnel technique, the inclination of the funnel being 60°, the pipe length 23 mm and the inner diameter 11 mm.

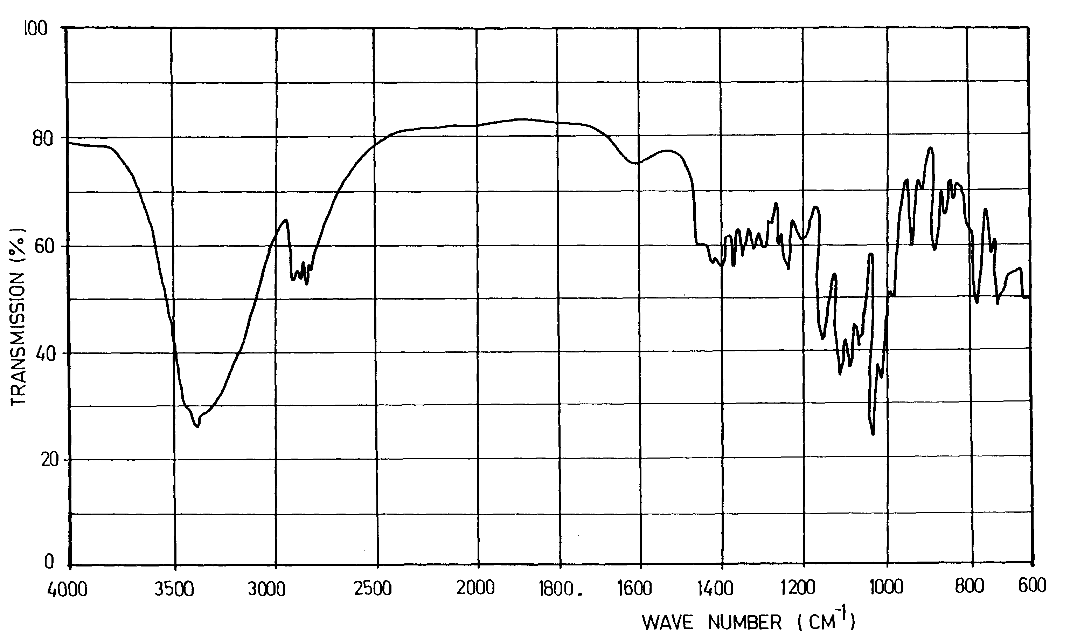

The infrared absorption of the lactitol monohydrate was measured by a Perkin-Elmer 398 spectrometer from a tablet having a composition of 1 g of lactitol monohydrate and 131 g of KBr. The infrared spectrum is shown in the drawing.

No more than 150 g of pure lactitol monohydrate on a dry substance basis dissolves in 100 ml of water at 25° C. Pure lactitol monohydrate crystals are colourless, odourless and transparent.

An accurate determination of the melting range of lactitol monohydrate can be most successfully carried out by introducing samples of milled lactitol into several capillary tubes and melting the open ends of the tubes before measuring. The measurements are carried out with a conventional melting point apparatus at different constant temperatures using one capillary tube per measurement until the extreme points of the melting range are found.

When determining the melting point, one must take into account that molten lactitol monohydrate has a high viscosity at its melting temperature, wherefore it takes time (even 2 minutes) before the sample is spread evenly on the walls of the capillary tube. Furthermore, the melt often contains bubbles caused by the liberation of crystal water, which remain in the melt for a long time.

In the process according to the above-mentioned European Patent 0039981, lactitol monohydrate anhydride is prepared by crystallizing lactitol within the temperature range from 10 to 50°C from a seeded lactitol solution obtained by hydrogenation and evaporated to a concentration between 70 and 85% or from a mother liquor obtained from the first crystallization step. This process can be used for the crystallization of lactitol only when the purity of lactitol in the feed solution is high, and since dihydrate may already be crystallized from pure lactitol solution, the crystallization of pure monohydrate is difficult if not impossible.

In the crystallization process according to the invention, crystallization temperatures (in the range from 80 to 30°C) are considerably higher than in the prior art process (from 50 to 10°C), whereby it is possible to crystallize lactitol monohydrate in at least four successive crystallization steps. With the present new process the total yield of lactitol monohydrate (see the crystallization series of Example 1, wherein the total yield is 97.6% on lactitol) is considerably higher than can be achieved with the prior art process (no more than 85% on lactitol).

The crystallization tests showed that if the crystallization is to occur in a controlled manner for obtaining a desired crystal size without a wide crystal size distribution, the crystallization should be effected in such a manner that the supersaturation of the mother liquor remains below 1.3 (preferably 1.2) with respect to lactitol throughout the crystallization. The supersaturation can be maintained within a desired range either by using a sufficiently long crystallization time or by measuring the dry substance content of the mother liquor with a refractometer. The supersaturation can be calculated from the dry substance content of the mother liquor and from the solubility curve of lactitol. The supersaturation (s) is defined as follows:

$$s = \frac{Cml \cdot (100 - Cml')}{Cml' \cdot (100 - Cml)}$$

Cml = measured dry substance content of mother liquor, % by weight
Cml'= solubility of lactitol in the mother liquor

Tests carried out and their results

Monohydrate anhydride ( Lacty-M, LCDE-31) partially restored during storage and having a melting range 97-103°C corresponding to 2% anhydration was cloddy and possessed a hygroscopicity substantially greater than that of the monohydrate (from Test 2 in Example 1). Water absorptions at 20°C after storage for 3 days at various relative humidities are shown in the following Table I.

3

## Table I. Hygroscopicity comparison

| f | Monohydrate (Example 1, Test 2) | Lacty-M LCDE-31 |
|---|---|---|
| 75% | 0.05 wt % | 0.2 wt % |
| 85% | 0.2 wt % | 0.5 wt % |
| 95% | 2.5 wt % | 3.3 wt % |

**f = relative humidity of ambient air, %.**

Drying tests were carried out on lactitol monohydrate in a conventional laboratory oven at a pressure of 1 bar. The samples were weighed and the degree of anhydration was calculated as a function of the drying time. Table II shows the degree of anhydration under varying drying conditions.

## Table II Anhydration tests

| Drying time (h) | Degree of anhydration (%) | | | | | | |
|---|---|---|---|---|---|---|---|
| | 1 20°C 0% | 2 40°C 25% | 3 60°C 15% | 4 70°C 10% | 5 80°C 5% | 6 90°C <5% | 7 105°C <5% |
| 0 | 0 | 0 | 0 | 0 | 0 | 0 | 0 |
| 1 | - | - | 2 | 4 | 8 | 34 | 78 |
| 2 | - | - | 4 | 10 | 26 | 86 | 100 |
| 4 | - | - | 6 | 20 | 78 | 100 | - |
| 24 | 0.08 | - | - | - | 98 | - | - |
| 55 | - | 0.3 | - | - | - | - | - |
| 72 | 4.1 | - | 86 | 96 | - | - | - |
| 121 | - | - | 94 | 100 | - | - | - |
| 142 | - | 0.4 | - | - | - | - | - |
| 144 | 12 | - | - | - | - | - | - |
| 219 | 21 | - | - | - | - | - | - |
| 338 | 38 | - | - | - | - | - | - |

1) sample of 10 g at 20°C when f = about 0%,
2) sample of 10 g at 40°C when f = about 25%,
3) sample of 200 g at 60°C when f = about 15%,
4) sample of 200 g at 70°C when f = about 10%,
5) sample of 200 g at 80°C when f = about 5%,
6) sample of 200 g at 90°C when f < 5%,
7) sample of 200 g at 105°C when f < 5%,
f = relative humidity of ambient air, %

The melting ranges of the partially anhydrated monohydrates formed in the test are 100-146°C (cf. Example 3).

On account of its excellent technical and physiological properties, the new lactitol monohydrate is particularly suitable as a substitute for sugar in diabetic, dietetic or tooth-friendly products. By combining lactitol monohydrate with other bulk or intense sweeteners, such as saccharin, Aspartame, Acesulfame K, Alitane, Sucralose, Stevioside or xylitol, a product highly resembling sugar and yet having a lower energy content and further being tooth-friendly can be prepared. Also this product is novel, and can be used instead of sugar e.g. in sugar products, confectionery, jams, bakery products, table-top sweeteners, cereals, desserts, chocolate, beverages, chewing gum and ice-creams, as well as in pharmaceutical and cosmetic products, such as toothpaste.

Example 1 Cooling crystallization

A four-step crystallization test sequence was carried out on lactitol monohydrate, starting from a filtered and de-ionised lactitol solution. The lactitol solution had been prepared from a lactose solution hydrogenated by the conventional technique.

All of the nine crystallization tests of this Example were carried out analogously with Test 1 which was performed in the following manner:

The crystallization was carried out according to the following steps: A lactitol solution having a purity of 98.3% lactitol in the dry matter was evaporated to 82.1% by weight at a temperature above 70°C, and 423 kg thereof was transferred into a crystallizer. The crystallizer was a conventional horizontal cylindrical batch-operated cooling crystallizer having a volume of 0.4 $m^3$ and provided with a mixer and a recycling water jacket whose temperature was controlled by means of a microprocessor. In the crystallizer, the temperature of the solution was adjusted to 70°C, whereafter the solution was seeded with ground lactitol monohydrate crystals. The seed crystal size was 0.02-0.05 mm, and the quantity thereof was 0.004% by weight on the lactitol in the batch. After the seeding, the mass was cooled in 16 hours down to 40°C, first slowly and ultimately more rapidly.

When the crystallization was complete the crystals were separated from the mother liquor with a conventional basket centrifuge wherein the crystals were also washed using 9.2% of water per obtained amount of crystal product. The centrifuged crystals were dried with a drum dryer using the conventional technique. The diameter of the cocurrent drum dryer used was 0.6 m, height 2.5 m and inclination about 1°; the speed of rotation was 3.5 rpm and the temperature of the drying air was 95°C. The feed rate of lactitol monohydrate was about 1.2 kg/min and the delay time about 30 minutes.

The performance conditions and results of the crystallization tests are presented in Tables III and IV hereinafter.

The total yield of lactitol monohydrate (four-step crystallization) was 97.6 %.

Table III Performance conditions of cooling crystallization tests

| Test | 1 | 2 | 3 | 4 | 5 | 6 | 7 | 8 | 9 |
|------|-----|-----|-----|-----|-----|-----|-----|-----|-----|
| Step | I | I | I | I | I | II | II | III | IV |
| a | 423 | 392 | 422 | 453 | 353 | 443 | 361 | 320 | 126 |
| b | 82.1 | 83,1 | 82.3 | 82.5 | 82.5 | 81.5 | 83.1 | 85.7 | 86,0 |
| c | 98,3 | 98,5 | 98.2 | 98.8 | 97.9 | 96.5 | 95,3 | 91,3 | 81,2 |
| d | 0,004 | 0.005 | 0.004 | 0.004 | 0 | 0.004 | 0,009 | 0,007 | 0,014 |
| e | 70 | 70 | 70 | 71 | 70 | 67 | 70 | 70 | 70 |
| f | 40 | 40 | 40 | 40 | 40 | 37 | ·40 | 40 | 40 |
| g | 16 | 16 | 32 | 16 | 32 | 15 | 17 | 40 | 110 |
| h | 9.2 | 11.1 | 9.5 | 9,4 | 7.8 | 14,2 | 13.8 | 19,0 | 19.5 |

| | |
|---|---|
| a | total amount of mass to be crystallized at the time of seeding, kg |
| b | dry matter content of mass at the time of seeding, % by weight |
| c | lactitol purity of mass, % by weight on dry matter |
| d | amount of seeds, % by weight on lactitol in the mass |
| e | seeding temperature, $^\circ$C |
| f | final temperature of crystallization, $^\circ$C |
| g | crystallization time, h |
| h | amount of washing water, % by weight per product crystal |

EP 0 456 636 B1

Table IV Results of cooling crystallization tests

| Test | 1 | 2 | 3 | 4 | 5 | 6 | 7 | 8 | 9 |
|------|---|---|---|---|---|---|---|---|---|
| Step | I | I | I | I | I | II | II | III | IV |
| a | 55.4 | 49.8 | 53.0 | 54.2 | 60.3 | 56.0 | 58.6 | 60.8 | 53.8 |
| b | 99.9 | 99.7 | 99.7 | 99.9 | 100 | 99.6 | 99.5 | 99.4 | 97.5 |
| c | 95.0 | 95.0 | 95.0 | 95.1 | 95.0 | 95.0 | 95.0 | 95.0 | 95.0 |
| d | 0.68 | 0.55 | 0.41 | 0.49 | 0.39 | 0.42 | 0.41 | 0.35 | 0.31 |
| e | 45 | 42 | 39 | 34 | 35 | 33 | 32 | 26 | 23 |
| f | 94-98 | 94-98 | 94-98 | 94-98 | 94-98 | 94-98 | 93-96 | 92-95 | 91-94 |

a crystal yield after drying, % by weight on lactitol

b lactitol purity of product crystal, % by weight on dry matter

c dry matter content of product crystal, % by weight

d average crystal size of product crystal, mm

e standard deviation from average size of product crystal, %

f melting range of product crystal, °C

Crystallization Example 1 is intended to illustrate the practicability of the novel process, but the crystallization may also be carried out by modifying it in a manner as required by normal effective production operation. Thus the crystallization may also be performed without adding seed crystals, i.e. by allowing the solution to form seeds spontaneously as in crystallization test 5. Further, the crystallization may be effected entirely or partially by evaporative crystallization as demonstrated in Example 2. The crystallization may also be carried out in a continuous operation as long as the temperature is maintained in the range 80°C-30°C and the supersaturation of the mother liquor is maintained below 1.3.

Example 2 Evaporation crystallization

Crystallization of lactitol monohydrate was performed starting from a lactitol solution prepared by hydrogenation (the same as in Example 1). The solution was evaporatively crystallized for 5 hours at 60°C, whereafter the crystals were separated from a slightly cooled mass, washed, and dried, as explained in the following.

The lactitol solution was concentrated in a conventional 0.4 m$^3$ evaporation crystallizer at 60°C at a pressure of about 180 mbar until the dry matter content of the solution was 80.9% by weight and there was approximately 30% of solution on the volume of the crystallizer, at which point the solution was seeded with lactitol monohydrate seed crystals. The amount of seed crystals was 0.008% by weight of the lactitol monohydrate content of the final batch, and the average size of the seed crystals was about 0.03 mm. After the seeding, more feed solution was supplied to the crystallizer, and the evaporation was continued at 59-65°C so that the dry matter content of the mother liquor was in the range 78-82% by weight.

After evaporating for 5 hours, the crystallizer was replete with a mass which was transferred into a cooling crystallizer and cooled from 62°C to 55°C in 10 hours, whereafter the crystals were separated from the mother liquor by centrifuging and dried as in Example 1. The crystal yield was 49.7% on lactitol. The purity of the lactitol monohydrate product was 99.7% on a dry matter basis, the dry matter content was 95.0% and the melting range 94.5-98°C.

Example 3 Anhydration of monohydrate

The lactitol monohydrate produced in Test 2 of Example 1 was dried at 20-105°C with drying air having a relative humidity of 0-25% for varying periods of time, whereby different partially anhydrated monohydrates were obtained. The melting range of the anhydrated monohydrates obtained is shown as a function of the degree of anhydration in Table V:

## Table V Melting ranges

| Ds | An | Mp' | Mp | |
|---|---|---|---|---|
| 95.00 | 0 | 94 | 98 | monohydrate |
| 95.15 | 3 | 100 | 105 | |
| 95.25 | 5 | 103 | 118 | |
| 96.10 | 22 | 113 | 128 | |
| 98.40 | 68 | 129 | 140 | |
| 99.10 | 82 | 138 | 143 | |
| 100.00 | 100 | 144 | 146 | anhydride |

```
Ds  = dry matter content of product, % by weight
An  = degree of anhydration, i.e. amount of removed
      crystal water, %
Mp' = starting point for melting, °C

     Mp  = melting point, °C
```

Like Example 1, Examples 2 and 3 are intended to illustrate the invention, but the crystallization can be carried out also by modifying it in a manner as required by normal effective production operation, as explained hereinabove.

Example 4 Lactitol plain chocolate

| | g |
|---|---|
| Cocoa butter | 165 |
| Cocoa liquor | 630 |
| Lactitol monohydrate | 719 |

Continuation of the Table on the next page

(continued)

|  | g |
| --- | --- |
| Acesulfame K | 2.3 |
| Vanillin | 0.3 |
| Lecithin | 6 |

Procedure: Conch. 17 hours at 50° C and 3 hours at 60° C.

Example 5 Lactitol milk chocolate

|  | g |
| --- | --- |
| Cocoa butter | 345 |
| Cocoa liquor | 195 |
| Milk powder, fat 26 % | 209 |
| Lactitol monohydrate | 789 |
| Acesulfame K | 1.4 |
| Vanillin | 0.3 |
| Lecithin | 6 |

Procedure: Conch. 20 hours at 50° C.

Example 6 Lactitol chewy toffee

|  | g |
| --- | --- |
| Lactitol monohydrate | 306 |
| Finmalt L (maltitol syrup) | 306 |
| Acesulfame K | 0.2 |
| Vegetable fat | 39 |
| Emulsifier (Glycerylmonostearate) | 3 |
| Gelatine | 12 |
| Water | 25 |
| Citric acid | 8 |
| Flavour, colour | 1.5 |

Procedure:

1. Mix lactitol monohydrate, Finmalt L, vegetable fat and emulsifier.
2. Heat to 120° C.
3. Add dissolved gelatine.
4. Add citric acid, flavour and colour.
5. Pull the mass 2-4 minutes.
6. Form the mass.

<u>Example 7</u> Lactitol gelatine jelly

|  | g |
|---|---|
| Lactitol monohydrate | 200 |
| Finmalt L (maltitol syrup) | 267 |
| Acesulfame K | 0.63 |
| Water | 50 |
| Gelatin 250 BL | 35 |
| Water | 70 |
| Citric acid (50 % solution) Flavour, colour as required | 5 |

Procedure:

1. Mix lactitol monohydrate, Finmalt L, Acesulfame K and water.
2. Heat to 116° C.
3. Cool to 90°C and add dissolved gelatine.
4. Add citric acid, flavour and colour.
5. Deposit into starch moulds.

<u>Example 8</u> Lactitol pectin jelly

```
                                       g
      Pectin (HM confectionery)    15
      Lactitol monohydrate         50
      Acesulfame K                  0.7
      Water                       200

      Sodium citrate                4
      Citric acid                   3.7
      Lactitol monohydrate        265
      Finmalt L (maltitol syrup)  630
      Citric acid (50 % solution)   8.5
      Flavours, colours             2.5
```

Procedure:

1. Homogenise pectin and lactitol monohydrate.
2. Add a solution of water, sodium citrate and citric acid.
3. Heat to 100° C.
4. Add a homogenised mixture of lactitol monohydrate, Acesulfame K, Finmalt L, flavours and colours.
5. Heat to 106-108°C.
6. Add citric acid.
7. Deposit into starch or plastic moulds.

Example 9 Lactitol gum-arabic pastille

|  | g |
|---|---|
| Gum arabic, 50 % solution | 400 |
| Lactitol monohydrate | 220 |
| Finmalt (maltitol syrup) | 107 |
| Acesulfame K | 1.0 |
| Water | 100 |
| Citric acid (50 % solution) | 10 |
| Flavour, colour | 2 |

Procedure:

1. Mix lactitol and Finmalt to the water.
2. Heat to 120°C.
3. Add heated solution to gum arabic solution.
4. Add acid, flavour and colour.
5. Deposit into starch moulds.
6. Dry 48-60 hours at 60°C.

Example 10 Lactitol hard candy

|  | g |
|---|---|
| Lactitol monohydrate | 368 |
| Finmalt L (maltitol syrup) | 200 |
| Acesulfame K | 0.4 |
| Water | 100 |
| Flavours, colour | 2 |

Procedure:

1. Heat sweeteners and water to 160-162°C.
2. Keep the mass 10 minutes in vacuum (0.8-0.9 ....).
3. Cool the mass and mix flavours and colours.
4. Form the mass.

Example 11 Lactitol strawberry jam

|  | g |
|---|---|
| Strawberries | 300 |
| Water | 300 |
| Pectin (Obi Violettband B) | 6 |
| Lactitol monohydrate | 500 |
| Citric acid (50 % solution) | 3 |
| Calcium citrate | 0.2 |
| Calcium lactate | 0.3 |
| Potassium sorbate | 1.7 |

Procedure:

1. Dry mix the pectin and 50 g of the lactitol.

2. Heat the fruit and water for few minutes.
3. Sprinkle the pectin/lactitol mixture into the fruit/-water mixture.
4. Bring to boil and keep boiling for a moment to dissolve the pectin completely.
5. Add remainder of the lactitol and boil for a little while.
6. Add the preservative and the calcium salts soluted in a small amount of water.
7. Boil until the weight of the batch is 1000 g or until desired solid content is reached.
8. Stop boiling and add acid solution.
9. Cool to 70°C stirring from time to time and pack.

Example 12 Lactitol biscuits

|  | g |
|---|---|
| Lactitol monohydrate | 95 |
| Fructose | 95 |
| Fat | 10 |
| Whole egg | 50 |
| Flour | 175 |
| Fibre (oat bran) | 30 |
| Sodium bicarbonate | 7.5 |
| Salt | 1.5 |
| Ginger | 1 |
| Water | 40 |

Procedure:

1. Cream fat with lactitol and fructose.
2. Mix eggs in one by one.
3. Shift together dry ingredients and add beating throughly.
4. Cool a few hours or overnight.
5. Bake in 170°C for about 11 minutes.

Example 13 Chocolate cake

|  | g |
|---|---|
| Lactitol monohydrate (milled) | 179.5 |
| Butter | 180.0 |
| Whole egg | 180.0 |
| Flour | 150.0 |
| Cocoa powder | 30.0 |
| Saccharin | 0.5 |

Procedure:

1. Dry mix the milled lactitol monohydrate with the saccharin.
2. Cream with the butter until light and fluffy.
3. Gradually beat in the eggs.
4. Fold in the flour and cocoa powder.
5. Deposit into a greased cake tin (16 cm diameter).
6. Bake for 60 minutes at 180° C.

Example 14 Fatless sponge cake

|  | g |
|---|---|
| Lactitol monohydrate (milled) | 89.3 |
| Eggs (separated) | 180 |
| Flour | 90 |
| Saccharin | 0.2 |

Procedure:

1. Dry mix the milled lactitol with the saccharin.
2. Whisk egg yolks with lactitol mixture until thick and creamy.
3. Whisk egg whites until firm and dry.
4. Fold egg white into egg yolk mixture.
5. Fold in flour.
6. Deposit into a greased and floured cake tin (16 cm diameter).
7. Bake for 40 minutes at 180° C.

Results:

Baked cake had golden colour and even crumb texture:

| Weight | 275 g |
|---|---|
| Height | 3.7 cm |
| Volume | 744 cm |
| Density | 0.37 g/ml |

Example 15 Ice cream

|  | g |
|---|---|
| Lactitol monohydrate | 140 |
| Butterfat | 80 |
| Skimmed milk powder | 110 |
| Water | 660 |
| Emulsified (stabiliser) (Grindstaad SE 33) | 8.1 |
| Aspartame | 0.4 |
| Colour (Bush Boake Allen Permucol egg yellow powder) | 0.06 |
| Vanilla flavour | 0.4 |

Procedure:

1. Dissolve the lactitol and skimmed milk powder in the water (reserving about 5 % to dissolve the aspartame).
2. Add the butterfat and emulsifier (stabiliser).
3. Pasteurise at 72° C for 10 minutes.
4. Homogenise.
5. Rapidly cool to 5° C and age overnight at 2 - 4° C.

6. Add colour, flavour and pre-dissolved aspartame.

7. Freeze to 100 % overrun.

Example 16 Frozen dessert

|  | g |
| --- | --- |
| Lactitol monohydrate | 100 |
| Fructose | 40 |
| Butterfat | 40 |
| Skimmed milk powder | 110 |
| Water | 700 |
| Emulsifier (stabiliser) (Grindstaad SE 33) | 9.3 |
| Aspartame | 0.24 |
| Colour (Bush Boake Allan Permucol egg yellow powder) | 0.06 |
| Vanilla flavour | 0.4 |

Procedure:

1. Dissolve the lactitol, fructose and skimmed milk powder in most of the water (reserving about 5 % to dissolve the aspartame).

2. Add the butterfat and emulsifier (stabiliser).

3. Pasteurise at 72° C for 10 minutes.

4. Homogenise.

5. Rapidly cool to 5° C and age overnight at 2 - 4 °C.

6. Add the colour, flavour and pre-dissolved aspartame.

7. Freeze to 100 % overrun.

Example 17 Sorbet

|  | g |
| --- | --- |
| Lactitol monohydrate | 250 |
| Strawberry puree | 150 |
| Gelatin 125° Bloom | 10 |
| Citric acid (50 %) | 4.0 |
| Aspartame | 0.8 |
| Colour (Hexacol Strawberry Red) | 0.3 |
| Strawberry flavour | 1.1 |
| Water | 584 |

Procedure:

1. Dissolve the lactitol in the water.

2. Add the gelatin and mix.

3. Pasteurise at 72° C for 10 minutes.

4. Rapidly cool to 5° C and age overnight at 2 - 4 °C.

5. Add strawberry puree, citric acid, colour and flavour.

6. Freeze to 65 % overrun.

Example 18 Table-top sweetener

(Equivalent sweetness to sugar)

|  | g |
|---|---|
| Lactitol monohydrate | 100 |
| Sodium saccharin | 0.23 |

Procedure:

Dry mix using a ribbon blade (or other suitable dry powder mixer) until a uniform dispersion is obtained.

Application:

Suitable for direct replacement of succrose in all applications.

Example 19 Table-top sweetener

(4 times as sweet as sugar)

|  | g |
|---|---|
| Lactitol monohydrate | 100 |
| Acesulfame K | 1.85 |

Procedure:

Dry mix using a ribbon blade (or other suitable dry powder mixer) until a uniform dispersion is obtained.

Applications:

Suitable for use in reduced caloric formulations where some bulk is needed.

Example 20 Table-top sweetener

(10 times as sweet as sugar)

|  | g |
|---|---|
| Lactitol monohydrate | 100 |
| Aspartame | 6.0 |

Procedure:

Dry mix using a ribbon blade (or other suitable dry powder mixer) until a uniform dispersion is obtained.

Applications:

Suitable for sprinkling or use in low caloric formulations where bulk is not required.

<u>Example 21</u> Drinking chocolate

|  | g |
|---|---|
| Lactitol monohydrate | 200 |
| Skimmed milk powder | 70 |
| Fat reduced cocoa powder | 12 |

Procedure:

Reconstitute with 708 g hot water (total 1000 g).

**Claims**

1. A crystalline lactitol monohydrate having lattice cell constants a = 7.815 ± 0.008 Á, b = 12.682 ± 0.008 Á, and c = 15.927 ± 0.008 Á, a melting range between 94 and 100°C, and a water content between 4.85 and 5.15%.

2. A crystalline lactitol monohydrate according to claim 1, **characterized** in that its flowability is better than 25 s/100 g, preferably better than 10 s/100 g, calculated by a funnel technique, the inclination of the funnel being 60°, the pipe length 23 mm and the inner diameter 11 mm.

3. A crystalline lactitol monohydrate according to claim 1, **characterized** in that its hygroscopicity is less than 0.2 % w/w humidity after 3 days at 20°C and 75 % relative humidity, and preferably less than 0.1 % w/w.

4. A process of preparing a crystalline lactitol monohydrate having lattice cell constants a = 7.815 ± 0.008 Å, b = 12.662 ± 0.008 Å, and c = 15.927 ± 0.008 Å; and a melting range between 94 and 100°C, by crystallizing from an aqueous solution which in addition to lactitol possibly contains impurities no more than 30 % on dry substance basis, **characterized** by evaporating the aqueous solution of lactitol to a concentration between 75 and 88 % by weight, seeding the evaporated solution at a temperature between 50 and 80°C, or allowing the solution to form seeds spontaneously at said temperature, optionally evaporating within a temperature range from 50 to 80°C for increasing the crystal content, cooling the resultant mixture of a temperature ranging between 30 and 60°C, subsequently separating the lactitol monohydrate crystals from the mother liquor, optionally washing, and subsequently drying with air having a temperature below 120°C, preferably between 60 and 100°C, and a relative humidity between 0 and 40 %, preferably between 5 and 20 %, for a time period less than 24 hours, preferably 5 to 50 minutes, whereby a final product is obtained having the water content between 4.85 and 5.15 %.

5. A process for preparing a crystalline lactitol monohydrate having lattice cell constants a = 7.815 ± 0.008 Å, b = 12.682 ± 0.008 Å and c = 15.927 ± 0.008 Å; and a melting range between 94 and 100°C, by crystallizing from an aqueous solution which in addition to lactitol possibly contains impurities no more than 30 % on dry substance basis, **characterized** by evaporating the aqueous solution of lactitol to a concentration between 80 and 88 % by weight at a temperature between 70 and 80°C, cooling the solution to a temperature between 65 and 75°C, seeding the solution, or allowing the solution to form seeds spontaneously at said temperature, subsequently cooling the resultant mixture slowly to a temperature within a range between 35 and 45°C, separating the lactitol monohydrate crystals from the mother liquor, optionally washing, and subsequently drying with air having a temperature below 120°C, preferably between 60 and 100°C, and a relative humidity between 0 and 40 %, preferably between 5 and 20 %, for a time period less than 24 hours, preferably for 5 to 50 minutes, the water content of the obtained product being between 4.85 and 5.15 %.

6. A process of preparing a crystalline lactitol monohydrate having lattice cell constants a = 7.815 ± 0.008 Å, b = 12.682 ± 0.008 Å and c = 15.927 ± 0.008Å and a melting range between 94 and 100°C, by crystallizing from a mother liquor obtained in a previous crystallization step and containing in addition to lactitol possibly impurities no more than 30 % on dry substance basis, **charac-terized** by evaporating said mother liquor to a concentration between 80 and 88 % by weight at a temperature between 70 and 80°C, cooling the solution to a temperature between 65 and 75°C, seeding the solution, or allowing the solution to form seeds spontaneously at said temperature, subsequently cooling the resultant mixture slowly to a temperature within a range between 35 and 45°C, separating the lactitol monohydrate crystals from the mother liquor, optionally washing, and subsequently drying with air having a temperature below 120°C, preferably between 60 and 100°C, and a relative humidity between 5 and 20 %, for a time period less than 24 hours, preferably for 5 to 50 minutes, the water content of the obtained product being between 4.85 and 5.15 %.

**7.** A process according to claim 4, 5 or 6, **characterized** in that the supersaturation of the mother liquor is maintained at a value below 1.3 (preferably 1.2) relative to lactitol throughout the crystallization.

**8.** A process according to claim 6, **characterized** in that said mother liquor is obtained from the previous, 2nd and 3rd crystallization step.

**9.** Use of the crystalline lactitol monohydrate according to claim 1, 2 or 3 as a bulk sweetener for the total or partial replacement of sucrose.

**10.** Use of the crystalline lactitol monohydrate according to claim 1, 2 or 3 in dietetic products, confectionery, bakery products, cereals, desserts, jams, beverages, chocolate, chewing gum and ice-cream, as well as in cosmetic products, and in the manufacture of pharmaceutical products, such as tooth paste.

**11.** A special sweetening agent resembling sucrose, **characterized** in that it is mainly composed of a crystalline lactitol monohydrate having lattice cell constants a = 7.815 ± 0.008 Á, b = 12.682 ± 0.008 Á, and c = 15.927 ± 0.008 Á, a melting range between 94 and 100°C, and a water content between 4.85 and 5.15%, and of a tooth-friendly sweetening agent such as saccharin or xylitol.

**Patentansprüche**

**1.** Kristallines Lactitolmonohydrat mit Zellgitterkonstanten von

a = 7,815 ± 0,008 Å, b = 12,682 ± 0,008 Å und
c = 15,927 ± 0,008 Å, einem Schmelzbereich zwischen 94 und 100°C und einem Wassergehalt zwischen 4,85 und 5,15 %.

**2.** Kristallines Lactitolmonohydrat gemäß Anspruch 1,

dadurch **gekennzeichnet**,
daß seine Fließbarkeit besser ist als 25 s/100 g, bevorzugterweise besser als 10 s/100 g, berechnet nach einer Trichtermethode, bei der die Neigung des Trichters 60°, die Rohrlänge 23 mm, und der Innendurchmesser 11 mm beträgt.

**3.** Kristallines Lactitolmonohydrat gemäß Anspruch 1,

dadurch **gekennzeichnet**,
daß seine Hygroskopizität weniger als 0,2 % w/w Feuchtigkeit nach 3 Tagen bei 20°C und 75 % relativer Feuchtigkeit, und bevorzugterweise weniger als 0,1 % w/w, beträgt.

**4.** Verfahren zur Herstellung eines kristallinen Lactitolmonohydrats mit Zellgitterkonstanten von a = 7,815 ± 0,008 Å, b = 12,682 ± 0,008 Å und c = 15,927 ± 0,008 Å, und einem Schmelzbereich zwischen 94 und 100°C durch Kristallisation aus einer wäßrigen Lösung, die zusätzlich zu Lactitol möglicherweise Verunreinigungen enthält von nicht mehr als 30 % auf Basis der Trockensubstanz,

dadurch **gekennzeichnet**,
daß die wäßrige Lactitol-Lösung auf eine Konzentration zwischen 75 und 88 Gew.-% eingedampft wird, die eingedampfte Lösung bei einer Temperatur zwischen 50 und 80°C angeimpft oder in diesem Temperaturbereich die spontane Keimbildung in der Lösung ermöglicht wird, die Lösung wahlweise zur Erhöhung des Kristallgehaltes im Temperaturbereich von 50 bis 80°C weiter eingedampft wird, die resultierende Mischung auf eine Temperatur im Bereich von 30 bis 60°C abgekühlt wird und anschließend die Lactitolmonohydrat-Kristalle von der Mutterlauge abgetrennt, wahlweise gewaschen, und anschließend mit Luft einer Temperatur von unter 120°C, bevorzugterweise zwischen und 60 und 100°C, und einer relativen Feuchtigkeit zwischen 0 und 40 %, bevorzugterweise zwischen 5 und 20 %, für eine Zeitdauer von weniger als 24 h, bevorzugterweise 5 bis 50 min, getrocknet werden, wodurch ein Endprodukt erhalten wird, das einen Wassergehalt zwischen 4,85 und 5,15 % aufweist.

**5.** Verfahren zur Herstellung eines kristallinen Lactitolmonohydrats mit Zellgitterkonstanten von a = 7,815 ± 0,008 Å, b = 12,682 ± 0,008 Å und c = 15,927 ± 0,008 Å und einem Schmelzbereich zwischen 94 und 100°C durch Kristallisation aus einer wäßrigen Lösung, die zusätzlich zu Lactitol möglicherweise Verunreinigungen von nicht mehr als 30 % auf Basis der Trockensubstanz enthält,

dadurch **gekennzeichnet**,
daß die wäßrige Lactitol-Lösung bei einer Temperatur zwischen 70 und 80°C auf eine Konzentration zwischen 80 und 88 Gew.-% eingedampft wird, die Lösung auf eine Temperatur zwischen 65 und 75°C abgekühlt wird, die Lösung angeimpft oder die spontane Keimbildung in der Lösung in diesem Temperaturbereich ermöglicht wird, die resultierende Mischung anschließend langsam auf eine Temperatur im Bereich zwischen 35 und 45°C abgekühlt wird, die Lactitolmonohydrat-Kristalle von der Mutterlauge abgetrennt, wahlweise gewaschen, und anschließend mit Luft einer Temperatur unterhalb von 120°C, bevorzugterweise zwischen 60 und 100°C, und einer relativen Feuchtigkeit zwischen 0 und 40 %, bevorzugterweise zwischen 5 und 20 %, für eine Zeitdauer von weniger als 24 h, bevorzugterweise für 5 bis 50 min, getrocknet werden, und der Wassergehalt des erhaltenen Produktes zwischen 4,85 und 5,15 % liegt.

**6.** Verfahren zur Herstellung eines kristallinen Lactitolmonohydrats mit Zellgitterkonstanten von a = 7,815 ± 0,008 Å, b = 12,682 ± 0,008 Å und c = 15,927 ± 0,008 Å und einem Schmelzbereich zwischen 94 und 100°C durch Kristallisation aus einer Mutterlauge, die in einem vorangegangenen Kristallisationsschritt erhalten wurde und zusätzlich zu Lactitol möglicherweise Verunreinigungen von nicht mehr al 30 % auf Basis der trockenen Substanz enthält,

dadurch **gekennzeichnet**,
daß die wäßrige Lactitol-Lösung bei einer Temperatur zwischen 70 und 80°C auf eine Konzentration zwischen 80 und 88 Gew.-% eingedampft wird, die Lösung auf eine Temperatur zwischen 65 und 75°C abgekühlt wird, die Lösung angeimpft oder die spontane Keimbildung in der Lösung in diesem Temperaturbereich ermöglicht wird, die resultierende Mischung anschließend langsam auf eine Temperatur im Bereich zwischen 35 und 45°C abgekühlt wird, die Lactitolmonohydrat-Kristalle von der Mutterlauge abgetrennt, wahlweise gewaschen, und anschließend mit Luft einer Temperatur unterhalb von 120°C, bevorzugterweise zwischen 60 und 100°C, und einer relativen Feuchtigkeit zwischen 5 und 20 %, für eine Zeitdauer von weniger als 24 h, bevorzugterweise für 5 bis 50 min, getrocknet werden, und der Wassergehalt des erhaltenen Produktes zwischen 4,85 und 5,15 % liegt.

**7.** Verfahren gemäß Anspruch 4, 5 oder 6,

dadurch **gekennzeichnet**,
daß die Übersättigung der Mutterlauge während der gesamten Kristallisation auf einem Wert unterhalb von 1,3 (bevorzugterweise 1,2) in Bezug auf Lactitol gehalten wird.

**8.** Verfahren gemäß Anspruch 6,

dadurch **gekennzeichnet**,
daß die Mutterlauge erhalten wird aus dem vorangegangenen, zweiten und dritten Kristallisationsschritt.

**9.** Verwendung des kristallinen Lactitolmonohydrats gemäß Anspruch 1, 2 oder 3 als Massensüßmittel für den gesamten oder teilweisen Ersatz von Sucrose.

**10.** Verwendung des kristallinen Lactitolmonohydrats gemäß Anspruch 1, 2 oder 3 in dietätischen Produkten, Süßwaren, Bäckereiprodukten, Cerealien, Desserts, Marmeladen, Getränken, Schokolade, Kaugummi und Eiscreme, sowie auch in kosmetischen Produkten und in der Herstellung von pharmazeutischen Produkten wie Zahnpasta.

**11.** Spezielles Süßmittel, das an Zucker erinnert,

dadurch **gekennzeichnet**,
daß es hauptsächlich zusammengesetzt ist aus kristallinem Lactitolmonohydrat mit Zellgitterkonstanten von a = 7,815 ± 0,008 Å, b = 12,682 ± 0,008 Å und

c = 15,927 ± 0,008 Å, einem Schmelzbereich zwischen 94 und 100°C und einem Wassergehalt zwischen 4,85 und 5,15 %, und aus einem zahnfreundlichen Süßmittel wie Saccharin oder Xylitol.

## Revendications

1. Monohydrate de lactitol cristallisé, dont la maille cristalline présente les constantes a = 7,815 ± 0,008 Å, b = 12,682 ± 0,008 Å, et c = 15,927 ± 0,008 Å, dont l'intervalle de fusion se situe entre 94°c et 100°C, et dont la teneur en eau vaut entre 4,85 % et 5,15 %.

2. Monohydrate de lactitol cristallisé, conforme à la revendication 1, **caractérisé** en ce que son aptitude à l'écoulement est meilleure que 25 s pour 100 g, cette caractéristique étant déterminée à l'aide d'un entonnoir dont l'angle d'inclinaison vaut 60° et dont le tuyau présente une longueur de 23 mm et un diamètre intérieur de 11 mm.

3. Monohydrate de lactitol cristallisé, conforme à la revendication 1, **caractérisé** en ce que son caractère hygroscopique est si faible qu'il absorbe moins de 0,2 % en poids et de préférence moins de 0,1 % en poids d'humidité au bout de 3 jours à 20°C et sous 75 % d'humidité relative.

4. Procédé de préparation d'un monohydrate de lactitol cristallisé, dont la maille cristalline présente les constantes a = 7,815 ± 0,008 Å, b = 12,682 ± 0,008 Å, et c = 15,927 ± 0,008 Å, et dont l'intervalle de fusion se situe entre 94°C et 100°C, par cristallisation à partir d'une solution aqueuse contenant éventuellement, en plus du lactitol, des impuretés ne représentant pas plus de 30 % des matières sèches, **caractérisé** en ce que l'on fait s'évaporer la solution aqueuse de lactitol jusqu'à une concentration située entre 75 et 88 % en poids, on ensemence la solution résultant de cette évaporation, à une température située entre 50°C et 80°C, ou bien on laisse la solution former spontanément des germes à cette température, on reprend éventuellement l'évaporation dans l'intervalle de température allant de 50°C à 80°C, pour augmenter la teneur en cristaux, on fait refroidir le mélange résultant jusqu'à une température située entre 30°C et 60°C, puis on sépare les cristaux de monohydrate de lactitol d'avec la liqueur-mère, on les lave éventuellement et on les fait ensuite sécher avec de l'air dont la température est inférieure à 120°C, de préférence située entre 60°C et 100°C, et dont le taux d'humidité relative vaut entre 0 et 40 %, de préférence entre 5 et 20 %, pendant un laps de temps inférieur à 24 heures, valant de préférence de 5 à 50 minutes, grâce à quoi l'on obtient un produit final dont la teneur en eau vaut entre 4,85 et 5,15 %.

5. Procédé de préparation d'un monohydrate de lactitol cristallisé, dont la maille cristalline présente les constantes a = 7,815 ± 0,008 Å, b = 12,682 ± 0,008 Å, et c = 15,927 ± 0,008 Å, et dont l'intervalle de fusion se situe entre 94°C et 100°C, par cristallisation à partir d'une solution aqueuse contenant éventuellement, en plus du lactitol, des impuretés ne représentant pas plus de 30 % des matières sèches, **caractérisé** en ce que l'on fait s'évaporer la solution aqueuse de lactitol jusqu'à une concentration située entre 80 et 88 % en poids, à une température située entre 70°C et 80°C, on refroidit la solution jusqu'à une température située entre 65°C et 75°C, on ensemence la solution ou on la laisse former spontanément des germes à cette température, puis on refroidit lentement le mélange résultant jusqu'à une température située entre 35°C et 45°C, on sépare les cristaux de monohydrate de lactitol d'avec la liqueur-mère, on les lave éventuellement et on les fait ensuite sécher avec de l'air dont la température est inférieure à 120°C, de préférence située entre 60°C et 100°C, et dont le taux d'humidité relative vaut entre 0 et 40 %, de préférence entre 5 et 20 %, pendant un laps de temps inférieur à 24 heures, valant de préférence de 5 à 50 minutes, grâce à quoi l'on obtient un produit final dont la teneur en eau vaut entre 4,85 et 5,15 %.

6. Procédé de préparation d'un monohydrate de lactitol cristallisé, dont la maille cristalline présente les constantes a = 7,815 ± 0,008 Å, b = 12,682 ± 0,008 Å, et c = 15,927 ± 0,008 Å, et dont l'intervalle de fusion se situe entre 94°C et 100°C, par cristallisation à partir d'une liqueur-mère obtenue au cours d'une étape précédente de cristallisation et contenant éventuellement, en plus du lactitol, des impuretés ne représentant pas plus de 30 % des matières sèches, **caractérisé** en ce que l'on fait s'évaporer ladite liqueur-mère jusqu'à une concentration située entre 80 et 88 % en poids, à une température située entre 70°C et 80°C, on refroidit la solution jusqu'à une température située entre 65°C et 75°C, on ensemence la solution ou on la laisse former spontanément des germes à cette température, puis on refroidit lentement le mélange résultant jusqu'à une température située entre 35°C et 45°C, on sépare les cristaux de monohydrate de lactitol d'avec la liqueur-mère, on les lave éventuellement et on les fait ensuite sécher avec de l'air dont la température est inférieure à 120°C, de préférence située entre 60°C et 100°C, et dont le taux d'humidité relative vaut entre 0 et 40 %, de préférence entre 5 et 20 %, pendant un laps de temps inférieur à 24 heures, valant de préférence de 5 à 50 minutes, grâce à quoi l'on obtient un produit final dont la teneur en eau vaut entre 4,85 et 5,15 %.

EP 0 456 636 B1

**7.** Procédé conforme à la revendication 4, 5 ou 6, **caractérisé** en ce que le degré de sursaturation de la liqueur-mère en lactitol est maintenu à une valeur inférieure à 1,3, de préférence inférieure à 1,2, pendant toute la durée de la cristallisation.

**8.** Procédé conforme à la revendication 6, **caractérisé** en ce que ladite liqueur-mère est obtenue au cours d'une première, seconde ou troisième étape de cristallisation.

**9.** Utilisation du monohydrate de lactitol cristallisé conforme à la revendication 1, 2 ou 3, en tant qu'édulcorant agissant dans la masse, destiné à remplacer totalement ou partiellement le saccharose.

**10.** Utilisation du monohydrate de lactitol cristallisé conforme à la revendication 1, 2 ou 3, dans des produits diététiques, des produits de confiserie, pâtisserie et boulangerie, des céréales, des desserts, des confitures, des boissons, du chocolat, des gommes à mâcher et des crèmes glacées, ainsi que dans des produits cosmétiques et dans la fabrication de produits pharmaceutiques, comme des pâtes dentifrices.

**11.** Agent édulcorant particulier, ressemblant au saccharose, **caractérisé** en ce qu'il est constitué principalement d'un monohydrate de lactitol cristallisé, dont la maille cristalline présente les constantes a = 7,815 ± 0,008 Å, b = 12,682 ± 0,008 Å, et c = 15,927 ± 0,008 Å, dont l'intervalle de fusion se situe entre 94°C et 100°C, et dont la teneur en eau vaut entre 4,85 % et 5,15 %, et d'un agent édulcorant sans danger pour les dents, comme de la saccharine ou du xylitol.